**Europäisches Patentamt**

(19) **European Patent Office**

**Office européen des brevets**

(11) Numéro de publication: **0 157 687**
**B1**

(12) **FASCICULE DE BREVET EUROPÉEN**

(45) Date de publication du fascicule du brevet:
08.06.88

(21) Numéro de dépôt: **85400506.3**

(22) Date de dépôt: **15.03.85**

(51) Int. Cl.⁴: **A 61 B 6/03**

(54) **Procédé de reconstruction d'image par tomodensitométrie.**

(30) Priorité: **16.03.84  FR 8404113**

(43) Date de publication de la demande:
**09.10.85 Bulletin 85/41**

(45) Mention de la délivrance du brevet:
**08.06.88 Bulletin 88/23**

(84) Etats contractants désignés:
**DE GB IT NL**

(56) Documents cité:
**DE-A-2 655 000**
**DE-A-2 708 604**
**FR-A-2 391 696**
**GB-A-1 577 046**

(73) Titulaire: **THOMSON- CGR, 13, square Max-Hymans, F-75015 Paris (FR)**

(72) Inventeur: **Tan, Siv- Cheng, THOMSON- CSF SCPI 173, bld Haussmann, F-75379 Paris Cedex 08 (FR)**
Inventeur: **Nguyen, Tri Hue, THOMSON- CSF SCPI 173, bld Haussmann, F-75379 Paris Cedex 08 (FR)**
Inventeur: **Benchimol, Claude, THOMSON- CSF SCPI 173, bld Haussmann, F-75379 Paris Cedex 08 (FR)**

(74) Mandataire: **Grynwald, Albert, THOMSON- CSF SCPI 19, avenue de Messine, F-75008 Paris (FR)**

## Description

L'invention concerne un procédé de reconstruction d'image par tomodensitométrie, permettant d'améliorer la résolution spatiale de l'image reconstruite, sans réarrangement des données et permettant de ce fait une reconstruction rapide de cette image.

L'évolution des tomodensitomètres est liée pour beaucoup à celle de l'ensemble de mesures constitué par la source de rayons X et les moyens de détection entraînés en rotation autour du patient pour relever les informations nécessaires à la reconstruction de l'image. On a ainsi distingué successivement plusieurs "générations" de tomodensitomètres. La première, dite à translation-rotation mettait en oeuvre une source de rayons X émettant un faisceau étroit en direction d'un seul détecteur. La source et le détecteur étaient solidaires d'un équipage mobile en translation, portés par le système tournant. Pour chaque position angulaire prédéterminée, l'équipage mobile se déplaçait transversalement par rapport à la coupe à imager, pour relever un ensemble de valeurs de mesure, appelés "vue". Dans un tel système tous les rayons ayant traversé la coupe à imager étaient, par construction, parallèles, pour une même vue. La seconde génération conservait le principe de translation-rotation mais avec un faisceau de rayons X en éventail de relativement faible ouverture et un multi-détecteur comportant un petit nombre de détecteurs adjacents. La demande de brevet allemand DE-OS-2 655 000 décrit un tel tomodensitomètre de deuxième génération. Enfin, les tomodensitomètres de troisième génération qui, à ce jour, constituent l'essentiel des appareils fabriqués, utilisent un faisceau de rayons X en éventail, d'ouverture plus importante et un multi-détecteur comportant un grand nombre de détecteurs adjacents capables de recevoir tous les rayons ayant traversé la coupe, pour chaque position angulaire de l'ensemble de mesure. Le mouvement de translation à chaque position angulaire est donc supprimé, ce qui permet d'acquérir toutes les données nécessaires à une reconstruction en un temps beaucoup plus faible. La demande de brevet français FR A-2 391 696 décrit un tel tomodensitomètre de troisième génération.

L'invention permet d'améliorer la résolution spatiale de l'image reconstruite dans n'importe lequel des types de tomodensitomètre décrits briévement ci-dessus.

Dans cet esprit, l'invention concerne un procédé de reconstruction d'image par tomodensitométrie, du type consistant à faire tourner, autour d'un centre de rotation et dans le plan d'une coupe à imager un ensemble de mesures comprenant une source de rayons X emettant à partir d'un foyer et des moyens de détection comprenant une pluralité de détecteurs adjacents, lesdits moyens de détection opérant sur une plage de détection définie par la pluralité des détecteurs pour détecter le rayonnement X non absorbé, à relever un nombre prédéterminé de vues comprenant chacune un échantillonnage à pas constant de N valeurs, représentatives de valeurs d'atténuation linéaire, élaborées par lesdits moyens de détection pour une position angulaire donnée dudit ensemble de mesure, lesdites vues étant relevées à pas angulaire constant et à appliquer auxdites vues une déconvolution et une rétro-projection, le centre de la plage de détection étant décalé du quart dudit pas d'échantillonnage par rapport à la droite passant par le centre de rotation de l'ensemble de mesure et le foyer de la source, caractérisé en ce qu'il consiste

- à acquérir les vues d'une image sur un tour complet dudit ensemble de mesure,
- à pondérer pour chaque vue lesdites valeurs d'atténuation linéaire déduites du signal délivré par chaque détecteur par un premier coefficient choisi,
- à élaborer pour chaque vue un nombre de valeurs intermédiaires équivalent au nombre de valeurs d'atténuation, lesdites valeurs intermédiaires étant obtenues chacune en faisant la somme de deux valeurs d'atténuation linéaire correspondant à des valeurs échantillonnées mesurées par deux détecteurs adjacents desdits moyens de détection et en pondérant cette somme par un second coefficient choisi et,
- à appliquer ladite déconvolution et ladite rétro-projection a chaque ensemble de valeurs d'atténuation pondérées et de valeurs intermédiaires, correspondant à chaque vue.

De préférence le pas angulaire est un sous-multiple d'un demitour ($\pi$) de l'ensemble de mesure. On pense cependant que cette condition n'est pas impérative, surtout lorsque ledit pas angulaire est faible, c'est-à-dire lorsqu'on utilise un grand nombre de vues pour reconstituer l'image.

Comme mentionné précédemment, cette définition de l'invention est valable quelque soit le type de tomodensitomètre. En particulier, dans le cas actuellement le plus fréquent d'un tomodensitomètre à faisceau en éventail et multidétecteur "larges", l'acquisition d'une vue se fait par lecture du multidétecteur à chaque position angulaire prédéterminée de l'ensemble de mesure (N étant le nombre de détecteurs ou "canaux" dudit multidétecteur). La plage de détection définie ci-dessus est bien entendu la face du multidétecteur orientée vers la source de rayons X à faisceau en éventail et le décalage entre le centre de cette plage et le centre de rotation est un décalage angulaire.

Il est à noter cependant que la justification mathématique qui va suivre du procédé de l'invention n'est rigoureuse que dans le cas de vues obtenues à partir de familles respectives de rayons parallèles. Le procédé a pourtant été expérimenté avec succès sur un tomodensitomètre de "troisième génération" à faisceau en éventail et multidétecteur "larges". On a constaté une amélioration très sensible de la résolution spatiale, spécialement dans la région centrale de l'image car on peut considérer que les rayons qui traversent la coupe à imager sont pratiquement parallèles au voisinage du centre de la coupe. C'est pourquoi la suite de la description fera référence, pour simplifier, a un multidétecteur "large" englobant pour chaque vue tout le champ de la coupe à imager, ledit multidétecteur étant représenté pour les besoins de la cause avec une structure rectiligne et recevant à chaque "vue" une famille de rayons parallèles provenant de moyens d'irradiation fictifs, non représentés. Actuellement aucun tomodensitomètre existant n'est évidemment conforme a une telle structure d'ensemble de mesure mais il suffit de savoir que le procédé est transposable tel quel sur un tomodensitomètre de "troisième génération".

L'invention sera mieux comprise et d'autres avantages de celle-ci apparaîtront à la lumière de la description qui va suivre du procédé conforme à l'invention, donnée en référence aux dessins annexés dans lesquels:

- la figure 1 représente schématiquement un multidétecteur irradié par une famille de rayons parallèles repérés dans un système d'axes orthonormés $(\theta_l, d)$ caractéristique d'une vue avec la représentation de la fonction d'absorption p, échantillonnée, correspondant à cette vue;

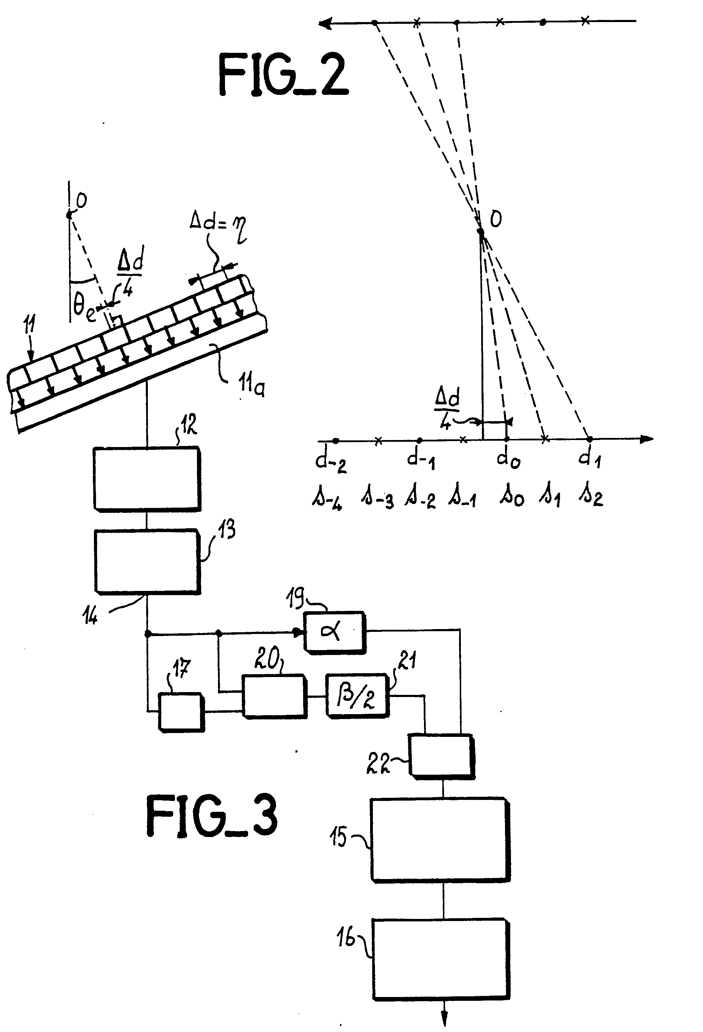

- la figure 2 représente les positions du multidétecteur pour deux vues décalées de $\pi$, les positions des centres des détecteurs réels étant repérées par des points et des positions intermédiaires correspondant à un échantillonnage double, $s_i$, étant repérées par des x; et
- la figure 3 est un schéma bloc d'un tomodensitomètre mettant en oeuvre les perfectionnements de l'invention.

Les procédés de reconstruction connus en tomodensitométrie mettent en oeuvre deux traitements, d'une part une Déconvolution et d'autre part une Rétroprojection. Dans les expressions de ces deux opérations, on prendra en compte la double discrétisation du système due, d'une part à l'échantillonnage de la fonction d'absorption par le fait que le signal est prélevé par un ensemble de détecteurs adjacents (on utilisera le terme "multidétecteur" pour désigner cet ensemble) et d'autre part aux positions angulaires $\theta_l$ de l'ensemble source-détecteurs auxquelles sont relevées les différentes vues nécessaires à la reconstruction.

La Déconvolution $D^\eta$ d'une fonction quelconque $f(\theta_l, d_i)$ s'exprime de façon classique:

$$D^\eta f(\theta_l, d_i) = \Sigma_j f(\theta_l, d_j) \cdot k(d_{i-j}) = g(\theta_l, d_i) \qquad (1)$$

le terme $\eta$ exprime le pas d'échantillonnage dû aux détecteurs $d_i$ soit $\eta = d_i - d_{i-1}$

On voit que la Déconvolution s'opère vue par vue, c'est-à-dire à $\theta_e$ constant ; k est une fonction de $d_i$, c'est le noyau qui caractérise la déconvolution.

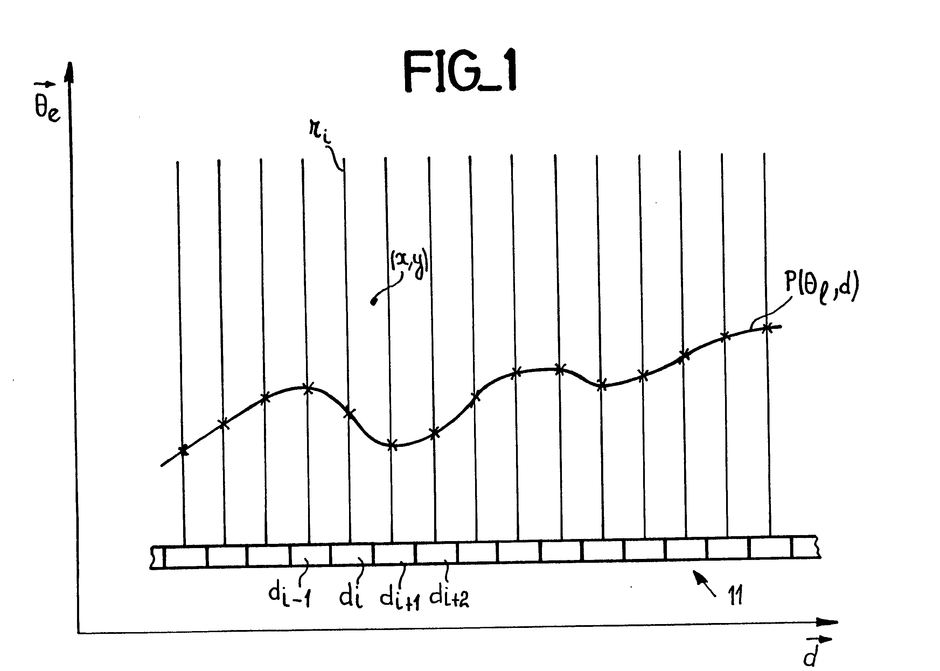

Avant d'aborder la rétroprojection, il convient d'introduire un opérateur supplémentaire I : l'opérateur d'interpolation. Soit en effet un point (x, y) quelconque de la coupe, auquel doit correspondre un pixel de l'image, après reconstruction. Pour une position angulaire donnée $\theta_l$ du multidétecteur 11 (voir figure 1), il se peut qu'aucun rayon $r_i$ venant frapper le centre d'un détecteur $d_i$ ne passe par le point (x, y). Il est donc usuel d'attribuer à ce point une valeur obtenue par interpolation. On peut par exemple associer au point (x, y) le rayon $r_i$ qui passe au plus près de celui-ci dans une vue donnée (c'est-à-dire la valeur mesurée au détecteur correspondant). On procède plus souvent à une "barycentration" entre les deux rayons situés de part et d'autre du point (x, y) ou entre un plus grand nombre encore de rayons voisins. Autrement dit, on fait une moyenne entre les valeurs mesurées par plusieurs détecteurs correspondant aux rayons voisins, cette moyenne étant pondérée par une fonction de la distance séparant le point (x, y) de chaque rayon. C'est dire que l'opérateur I peut avoir des expressions mathématiques très différentes suivant le choix du constructeur de tomodensitomètre. Il est à noter que la justification du procédé selon l'invention suppose que l'opérateur soit linéaire et symétrique. La définition de la linéarité d'une fonction ou d'un opérateur est bien connue. En outre, on dit qu'un opérateur d'interpolation est symétrique si, pour toute fonction discrétisée et sa fonction symétrique, auxquelles on applique l'opérateur, les fonctions interpolées de ces deux fonctions symétriques sont aussi symétriques. A titre d'exemple, les opérateurs d'interpolation classiques qui ont été décrits brièvement ci-dessus sont linéaires et symétriques.

Soit $\tilde{g} = Ig$, g étant la fonction déconvoluée, définie ci-dessus (relation (1)). La réctprojection $[R^\eta g]$ (x, y), autrement dit R appliquée à g et calculée au point (x, y) s'exprime classiquement de la façon suivante

$$[R^\eta g] (x, y) = \Sigma_l \tilde{g}(\theta_l, x \cos \theta_l + y \sin \theta_l) \qquad (2)$$

On rappelle d'autre part que la fonction d'absorption du système $p(\theta, d)$ représente, pour chaque position angulaire $\theta_l$ du multidétecteur (c'est-à-dire pour chaque "vue") la variation de l'absorption des rayons X dans le champ d'observation, repérée dans un repère orthonormé d'axes $\theta_e$, d (voir figure 1). De par la structure du multidétecteur, on ne connaît que $p(\theta_e, d_i)$ pour chaque vue d'angle $\theta_e$, c'est-à-dire une famille d'échantillons repérés par des x sur la figure 1.

Si f était la fonction d'absorption définie ci-dessus, alors la rétroprojection appliquée à la fonction f déconvoluée serait la reconstruction recherchée. Cependant, les développements qui suivent sont valables pour toute fonction f de et $d_i$.

Soit $L^\eta$ l'opérateur global défini par:

$L^\eta f = [R^\eta g](x,y)$

$\underline{g}$ étant relié à $\underline{f}$ par la relation (1). Cet opérateur sera appelé "operateur de reconstruction". On sait par ailleurs que les informations nécessaires pour reconstruire une image peuvent s'acquérir sur un demi-tour ou sur un tour complet de l'ensemble sourcemultidétecteur.

Soient $L^{\eta}_{\pi}f$ et $L^{\eta}_{2\pi}f$ les opérateurs de reconstruction correspondants, associés au pas d'échantillonnage $\eta$ du multidétecteur. Une des conditions de mise en oevre de l'invention est que

$\Delta\theta = \theta_l - \theta_{l-1}$

soit tel que $|\frac{\pi}{\Delta\theta}| = L$, entier. $\underline{L}$ est donc le nombre de vues sur un demi tour.

Dans ces conditions: FIG0/20 $\tilde{g}(\theta_l, x \cos\theta_l + y \sin\theta_l)$

FIG0/20 $\tilde{g}(\theta_l, x \cos\theta_l + y \sin\theta_l)$

Dans la pratique, c'est-à-dire lorsque $\underline{f}$ est la fonction d'absorption, on sait que:

$L^{\eta}_{2\pi} f = 2 L^{\eta}_{\pi}f$

Autrement dit, l'image reconstruite à partir des informations recueillies sur un tour complet est la même que celle qui est reconstruite à partir des informations recueillies sur un demi-tour.

L'invention a pour but d'exploiter cette redondance pour améliorer la résolution spatiale de l'image. Pour cela, on démontre que les opérateurs $L_{\pi}$ et $L_{2\pi}$ possèdent les propriétés intrinsèques (quelle que soit $\underline{f}$) suivantes:

1°) $L_{\pi}$ et $L_{2\pi}$ sont des opérateurs linéaires si l est un opérateur linéaire.

2°) en posant : $\bar{f}(\theta_l, d_i) = f(\theta_{l+L}, -d_i)$

on peut vérifier une autre propriété reliant $L_{\pi}$ et $L_{2\pi}$:

$L^{\eta}_{\pi}f = L^{\eta}_{\pi}f + L^{\eta}_{\pi}\bar{f} = L^{\eta}_{\pi}(f + \bar{f})$ \hfill (3)

pour autant que l soit linéaire et symétrique.

On cherche à exploiter les données fournies par les détecteurs avec un échantillonnage $\eta$, sur un tour complet (qui fournirait normalement une reconstruction $L^{\eta}_{2\pi}$) pour obtenir en fait une image correspondant à une reconstruction sur un demi-tour à partir de données qui seraient obtenues par un multidétecteur de pas d'échantillonnage $h = \eta_2$, soit $L^{h}_{\pi}$.

Pour cela, considérons le decalage mentionné ci-dessus du centre du multidétecteur par rapport à la projection du centre de rotation O sur celui-ci, les échantillons $s_i$ d'une vue doublement échantillonnée se définissent par la relation:

$s_i = ih + \frac{h}{2}$
et $s_i - s_{i-1} = h$

On rappelle aussi que les détecteurs réels $d_j$ sont reliés au pas $\eta$ par la relation:

$d_j = j\eta + \frac{\eta}{4}$

c'est-à-dire $d_j = 2jh + \frac{h}{2}$

$d_j = s_{2j}$

La suite $d_j$ se déduit donc de la suite $s_i$ par un prélèvement d'un échantillon sur deux.

Soit $p(\theta_l, d_j)$ la fonction d'absorption échantillonnée au pas $\eta$ du multidétecteur, il s'agit donc de l'ensemble des valeurs d'absorption déduites des lectures successives du multidétecteur.

Soit la fonction $\underline{f}$ suivante, d'échantillonnage de pas $\underline{h}$, définie par la double relation:

$f(\theta_l, s_{2i}) = \alpha \, p(\theta_l, d_i)$ \hfill (4a)
$f(\theta_l, s_{2i+1}) = \frac{\beta}{2} p(\theta_l, d_i) + p(\theta_l, d_{i+1})$ \hfill (4b)

En introduisant $\bar{f}$ d'après la définition qui précède, on peut vérifier que:

$(f + \bar{f})(\theta_l, s_i) = \alpha p(\theta_l, s_i) + \frac{\beta}{2}[p(\theta_l, s_{i+1}) + p(\theta_l, s_{i-1})]$

Par conséquent, si on pose de façon plus générale:

$q(\theta, s) = \alpha p(\theta, s) + \frac{\beta}{2}[p(\theta, s+h) + p(\theta, s-h)]$ \hfill (5)

On constate que:
$(f + \bar{f})(\theta_l, s_i) \equiv q(\theta_l, s_i)$.

Autrement dit, $(f + \bar{f})(\theta_l, s_i)$ n'est rien d'autre qu'une valeur échantillonnée de $q(\theta, s)$. Donc:

$L^{h}_{\pi}(f + \bar{f}) = L^{h}_{\pi}q$

Or d'après la relation (3)

$L^{h}_{\pi}(f + \bar{f}) = L^{h}_{2\pi}f$

d'où $L^{h}_{2\pi}f = L^{h}_{\pi}q$

Le but fixé, c'est-à-dire relier $L^{h}_{2\pi}f$ à $L^{h}_{\pi}p$ est presque atteint dans la mesure où $\underline{q}$ peut se déduire de la fonction d'absorption $\underline{p}$. En effet, si on prend la transformée de Fourier de la relation (5), à $\theta$ constant:

$\hat{q}(\theta, \nu) = H(\nu) \cdot \hat{p}(\theta, \nu)$ \hfill (6)

ou $\hat{q}$ et $\hat{p}$ sont respectivement les transformées de Fourier à $\theta$ constant de q et p
et où

$H_{(\nu)} = \alpha_T \beta \cos \pi \frac{\nu}{\nu c}$

$\nu c = \frac{1}{2h}$ est la fréquence de Nyquist associée à l'échantillonnage de pas $\underline{h}$.

(6) exprime donc que q est une "version filtrée" de p. Autrement dit, dans le domaine spatial q est le produit de convolution de p par un noyau $k_1$, $k_1$ étant la transformée de Fourier inverse de H.

Comme la convolution est commutative,
$L^{h}_{\pi}q = L^{h}_{\pi}p$
ou L est l'homologue de L avec un noyau de déconvolution k' tel que k' = k * k$_1$

 * étant le signe de l'opération de convolution.

Autrement dit, il suffit de choisir k (relation (1)) en sachant que le résultat final crrespondra en fait à une déconvolution de noyau k'. Si on applique l'invention à un tomodensitomètre connu de noyau donné, il suffit donc de modifier le noyau de déconvolution selon ce qui précède. Pour le faire, on dispose d'un grand nombre de possibilités car k$_1$ peut aussi être ajusté par un choix convenable de α et β.

Parmi les différentes possibilités dans le choix des paramètres α et β, on a pu vérifier une notable amélioration de la résolution spatiale en choisissant α ≠ β. En résumé, la mise en oeuvre de l'invention consiste essentiellement à modifier les valeurs représentatives de l'absorption, déduites des lectures successives des moyens de détection conformément à la double relation (4) en procédant à une acquisition des vues sur un tour complet.

La relation (4a) signifie simplement qu'on pondère par un premier coefficient α chaque valeur d'atténuation déduite du signal délivré par chaque détecteur d$_i$ (ou chaque position de détecteur dans le cas d'un tomodensitomètre à détecteur unique) dans chaque vue d'angle θ$_l$ et la relation (4b) signifie qu'on élabore dans chaque vue un nombre équivalent de valeurs intermédiaires en faisant chaque fois la somme de deux valeurs d'atténuation correspondant à des détecteurs adjacents (où à des positions de détecteur voisines) et en pondérant cette somme par un second coefficient β.

Dans le cas où on choisit α = β = 1, chaque valeur intermédiaire est donc obtenue en faisant tout simplement la moyenne de deux valeurs d'atténuation voisines.

En se référant à la figure 3, on a représenté les principaux sous-ensembles d'un tomodensitomètre équipé des perfectionnements conformes à l'invention. Les moyens de détection sont ici représentés sous forme d'un multidétecteur 11 comportant N détecteurs adjacents, le pas des détecteurs étant Δ d = η. Pour les raisons indiquées précédemment, le multidétecteur a été représenté sous forme d'un boîtier rectiligne mais il est bien connu que dans le cas d'un tomodensitomètre utilisant un faisceau de rayons X en éventail, le boîtier du multidétecteur pourra être courbe pour que tous les détecteurs d$_i$ soient agencés suivant un arc de cercle géométriquement centré sur le foyer de la source de rayons X, non représentés. L'ensemble de mesure comportant la source et le multidétecteur est entraîné en rotation dans le plan d'une coupe à imager. Le centre de rotation est O. On rappelle que le centre du multidétecteur est décalé de $\frac{\Delta d}{4}$ par rapport à la projection du centre O sur le multidétecteur et que le pas angulaire des prises de "vues" est tel que $|\frac{\pi}{\Delta\theta}| = L$, entier. Une vue est obtenue ici par lecture de tous les détecteurs du multidétecteur 11 pour un angle θ$_l$ donné. Sur la plupart des tomodensitomètres, il existe des moyens de réglage précis de la position du multidétecteur par rapport à la source. Dans la plupart des cas, ce "vernier" est ajusté pendant la phase de mise au point de l'appareil pour annuler aussi précisément que possible tout décalage entre le multidétecteur et la source. Les mêmes moyens peuvent être utilisés pour provoquer le décalage statique du multidétecteur, recherché dans le cadre de l'invention. La figure 3 décrit sous forme de schéma-bloc fonctionnel les moyens spécifiques de l'invention (selon un mode de réalisation possible) et les parties essentielles de l'ordinateur associé au multidetecteur 11. Les valeurs échantillonnées lues aux sorties du multidétecteur, au moyen par exemple d'un circuit de portes analogiques 11a, sont transformées en informations numériques grâce à un convertisseur Analogique-Numérique 12 puis traitées dans un préprocesseur 13, classique, effectuant les opérations habituelles de calibration et de transformation logarithmiques. Les informations ainsi transformées sont les valeurs d'atténuation linéaire. Chaque "vue" en comporte autant que le nombre de détecteurs du multidétecteur, c'est-à-dire N. De façon bien connue, le multidétecteur est lu chaque fois séquentiellement d'une extrémité à l'autre de sorte que les valeurs d'atténuation linéaire se succèdent à la sortie 14 du préprocesseur pour être traitées en temps réel dans un autre processeur de l'ordinateur comportant essentiellement une section 15 agencée ou programmée pour appliquer une deconvolution et une section 16 agencée ou programmée pour appliquer une retroprojection. Entre le préprocesseur 13 et les moyens de déconvolution, on a intercalé, selon l'invention, un registre 17 dont l'entrée est reliée à la sortie 14. Cette dernière est elle-même reliée à un multiplicateur numérique 19 agencé pour appliquer le coefficient α et à une entrée d'un sommateur numérique 20 dont l'autre entrée est reliée à la sortie du registre 17. La sortie du sommateur 20 est elle-même reliée à un autre multiplicateur numérique 21 agencé pour appliquer un coefficient β. Les sorties des deux multiplicateurs 19 et 21 sont reliés aux deux entrées d'un multiplexeur 22 dont la sortie alimente la section de déconvolution 15.

Ainsi, grâce au registre 17, on dispose à chaque instant de valeurs d'atténuation linéaire correspondant à deux détecteurs voisins. Les informations numériques représentatives de ces valeurs d'atténuation sont ensuite combinees conformément à la double relation (4a, 4b) grâce aux circuits 19, 20, 21. Chaque vue donne lieu ainsi à 2 N valeurs appliquées à la section de déconvolution. Bien entendu, toutes ces opérations peuvent aussi être exécutées par un simple sous-programme de l'ordinateur remplaçant tous les circuits de logique cablés qui ont été décrits entre le convertisseur Analogique-Numérique 12 et la section de Déconvolution 15.

La pondération de N valeurs d'atténuation par

le coefficient α donne N valeurs. En revanche l'obtention des valeurs intermédiaires à partir, chaque fois, de deux détecteurs voisins oblige de considérer une valeur de mesure fictive, supplémentaire, d'un côté du multidétecteur qui dépend du sens de décalage de ce dernier, pour obtenir N valeurs intermédiaires. Dans la pratique, il n'est nullement nécessaire d'ajouter un détecteur supplémentaire à l'ensemble de mesure car on peut considérer que l'absorption sur le bord de la coupe à imager est toujours nulle. Par conséquent, pour obtenir cette valeur intermédiaire extrême, il suffit de multiplier par $\frac{\beta}{2}$, la valeur d'absorption linéaire correspondant au détecteur réel extrême.

## Revendications

1. Procédé de reconstruction d'image par tomodensitométrie, du type consistant à faire tourner, autour d'un centre de rotation et dans le plan d'une coupe à imager, un ensemble de mesure comprenant une source de rayons X émettant à partir d'un foyer et des moyens de détection (11) comprenant une pluralité (N) de détecteurs adjacents, lesdits moyens de détection opérant sur une plage de détection définie par la pluralité des détecteurs pour détecter le rayonnement X non absorbé, à relever un nombre prédéterminé de vues comprenant chacune un échantillonnage à pas constant de N valeurs, représentatives de valeurs d'atténuation linéaire, élaborées par lesdits moyens de détection pour une position angulaire donnée dudit ensemble de mesure, lesdites vues étant relevées à pas angulaire constant et à appliquer auxdites vues une déconvolution (15) et une rétroprojection (16), le centre de la plage de détection étant décalé du quart dudit pas d'échantillonnage par rapport à la droite passant par le centre de rotation (O) de l'ensemble de mesure et le foyer de la source ,

caractérisé en ce qu'il consiste - à acquérir les vues d'une image sur un tour complet dudit ensemble de mesure,

- à pondérer (19) pour chaque vue lesdites valeurs d'atténuation linéaires déduites du signal délivré par chaque détecteur par un premier coefficient choisi (α),

- à élaborer pour chaque vue un nombre de valeurs intermédiaires equivalent au nombre de valeurs d'atténuation, lesdites valeurs intermédiaires étant obtenues chacune en faisant la somme (20) de deux valeurs d'atténuation lineaire correspondant à des valeurs echantillonnées mesurées par deux détecteurs adjacents desdits moyens de détection et en pondérant (21) cette somme par un second coefficient choisi ($\frac{\beta}{2}$), et

- à appliquer ladite déconvolution et ladite rétroprojection à chaque ensemble de valeurs d'atténuation pondérées et de valeurs intermédiaires, correspondant à chaque vue.

2. Procédé de reconstruction d'image selon la revendication 1, caractérisé en ce que le pas angulaire précité est un sous-multiple d'un demi-tour dudit ensemble de mesure.

## Patentansprüche

1. Verfahren zur Bildrekonstruktion durch Tomodensitometrie nach einer Weise, die darin besteht, daß eine Meßanordnung ein Rotationszentrum in der Ebene eines durch Bild aufzunehmenden Schnittes umläuft, welche eine aus einem Brennpunkt heraus emittierende Röntgenstrahlquelle sowie Anzeigemittel (11) mit einer Anzahl (N) einander benachbarter Detektoren umfaßt, wobei die Anzeigemittel über einen Ermittlungsbereich arbeiten, der durch die Anzahl der zur Anzeige der nichtabsorbierten Röntgenstrahlung dienenden Detektoren definiert ist; daß eine vorgegebene Anzahl von Aufnahmen gemacht wird, wobei jede aus einer Probenahme von H Werten bei konstanter Schrittfolge besteht, welche repräsentativ für die linearen Abschwächungswerte sind, die von den genannten Anzeigemitteln für eine gegebene Winkellage der Meßanordnung ermittelt werden, und die besagten Aufnahmen bei konstanter Winkelschrittfolge aufgenommen werden; und daß auf die Aufnahmen eine Dekonvolution (15) und eine Retroprojektion (16) angewendet wird, wobei das Zentrum des Ermittlungsbereiches um ein Viertel der genannten Schrittweite gegenüber der durch das Rotationszentrum (O) der Meßanordnung und den Brennpunkt der Quelle gehenden Geraden verschoben wird, dadurch gekennzeichnet, daß das Verfahren darin besteht, daß:

- die Aufnahmen eines Bildes über einen vollen Umlauf der Meßanordnung genommen werden,

- die linearen Abschwächungswerte, welche aus dem Signal jedes Detektors abgeleitet werden, durch einen ersten ausgewählten Koeffizienten (α ) gewichtet (19) werden

- für jede Aufnahme eine Anzahl von Zwischenwerten ermittelt wird, welche der Anzahl der Abschwächungswerte äquivalent ist, wobei die Zwischenwerte durch Summierung (20) der beiden linearen Abschwächungswerte gewonnen werden, die den von zwei benachbarten Detektoren der Meßanordnung gemessenen Pobenwerten entsprechen und die Summe mittels eines zweiten ausgewählten Koeffizientens ($\frac{\beta}{2}$) gewichtet wird, und

- die Dekonvolution und Retroprojektion auf jede Gesamtheit gewichteter Abschwächungswerte und Zwischenwerte angewendet wird, die jeder Aufnahme zugeordnet sind.

2. Verfahren zur Bildrekonstruktion nach Anspruch 1, dadurch gekennzeichnet, daß der Winkelschritt ein Untervielfaches des halben Umlaufs der Meßanordnung beträgt.

## Claims

1. Method for reconstructing pictures by tomodensitometry of the type consisting: of rotating a measuring device comprising an X-ray source emitting from a focus and detection means (11) comprising a plurality (N) of adjacent detectors around an his of rotation and within a plane of a section to be imaged, the said detection means operating across a detection range defined by a plurality of detectors for detecting the non-absorbed X-rays; of recording a predetermined number of views each comprising the sampling of N values at constant step representative for the linear attenuation values generated by the said detection means for a given angular position of the measuring device, the said views being taken at constant angular step; and of applying a deconvolution (15) and a retroprojection (16) to the views , the center of the detection range being displaced by a quarter of the said sampling step relative to a straight line passing through the center of rotation (O) of the measuring device and the focus of the source, characterized in that the method consists in:

- taking the views of a picture during a complete revolution of the measuring device
- weighting (19) for each view the said linear attenuation values of the signal delivered by each detector by a first selected coefficient ($\alpha$)
- generating for each view a number of intermediary values equivalent to the number of attenuation values, these intermediary values being obtained each by calculating the sum (20) of two linear attenuation values corresponding to the sample values measured by two adjacent detectors of the measuring device and weighting (21) each sum by a second selected coefficient ($\beta$), and
- applying the said deconvolution and retroprojection to each group of weighted attenuation values and intermediary values, corresponding to each record.

2. A method for reconstructing pictures according to claim 1, characterized in that the said angular step is a sub-multiple of a demi-revolution of the measuring device.

FIG_1

FIG_2

FIG_3

0 157 687